Europäisches Patentamt

European Patent Office

Office européen des brevets

(11) Publication number: **0 324 603**
A2

# (12) EUROPEAN PATENT APPLICATION

(21) Application number: 89300229.5

(22) Date of filing: 11.01.89

(51) Int. Cl.⁴: **G 01 N 33/543**
G 01 N 33/545, G 01 N 33/548

(30) Priority: 11.01.88 US 142013

(43) Date of publication of application:
19.07.89 Bulletin 89/29

(84) Designated Contracting States:
AT BE CH DE ES FR GB GR IT LI LU NL SE

(71) Applicant: GENERAL BIOMETRICS, INC.
P.O. Box 308
Bountiful Utah 84010 (US)

(72) Inventor: Golden, Carole Ann
2 White Oak Elon College
Alamance County North Carolina 27244 (US)

Wilson, Albert Jerome
4600 South 2850 West No. 195, West Valley City
Salt Lake County Utah 84119 (US)

Black, MelRee K.
2 White Oak Elon College
Alamance County North Carolina 27244 (US)

Kuts, Richard M.
18 Swan Sea Lane Pleasant Hill
Contra Costa County California 94523 (US)

(74) Representative: Ellis-Jones, Patrick George Armine et al
J.A. KEMP & CO. 14 South Square Gray's Inn
London WC1R 5EU (GB)

(54) Improved solid phase enzyme immunoassay test apparatus and process for detection antibodies.

(57) A method of preparing a stick component for a solid phase enzyme immunoassay system or kit is disclosed, which comprises heat bonding a porous nitrocellulose membrane to a chemically neutral plastic backing member comprising a plurality of openings, which backing member extends beyond the nitrocellulose membrane at its upper end when the two are bonded together to form a stick for handling, and applying a specific purified antigen dot to the nitrocellulose through at least one of the openings.

Also disclosed are a solid phase immunoassay system or kit comprising a stick component prepared by this method, and an assay method which involves using the stick component for the visual detection of specific antibodies in a clinical sample.

EP 0 324 603 A2

## Description

# IMPROVED SOLID PHASE ENZYME IMMUNOASSAY TEST APPARATUS AND PROCESS FOR DETECTION ANTIBODIES

## BACKGROUND OF THE INVENTION

### Field of the Invention

The present invention relates to immunochemical systems and processes for determining the presence of antibodies or antigens in a clinical sample and relates most closely to a procedure known as enzyme-linked immunosorbent assay (ELISA).

### Prior Art

In the science of immunology, heretofore a number of procedures have been developed for disease detection, many of which are based on the understanding that an antigen is a substance that can induce a detectible immune response when introduced within an animal. Responsive to that antigen introduction the body produces an antibody that is a protein with such antibody having the ability to combine with the antigen that stimulated its production. The body response to such antigen or antibody can therefore be determinative of whether the animal is suffering or has suffered from a particular disease or malady. With the identification of the presence of antibodies in a person's blood serum it can be concluded that an individual has been exposed to a particular type of antigen. European patent application 87306757.4 entitled "Solid Phase Enzyme Immunoassay Test Apparatus and Process for Detecting Antibodies" filed 30th July 1987 recognizes this medical fact and provides apparatus and a process for disease detection.

The above-cited patent application of the present inventors sets out in eleven categories certain tests for detection of antigens and antibodies in clinical practice, specifically: 1) immunodiffusion; 2) electrophoresis and immunoelectrophoresis; 3) immunochemical and physicochemical methods; 4) radioimmunoassay; 5) immunohistochemical techniques; 6) agglutination; 7) complement fixation; 8) immunofluorescence; 9) precipitation; 10) viral neutralization, and 11) ELISA. This earlier patent application then discusses a number of procedures and devices heretofore involved with antibody or antigen detection, each to produce some visual record for analysis. Similar to certain of these cited references, both the earlier and present application utilize nitrocellulose as a preferred support.

Where membranes of nitrocellulose are currently the most widely used transfer medium for protein blotting in that they are relatively cheap, simple to use and have a long shelf life of approximately twelve months at four degrees centigrade, the securing of a nitrocellulose support to a backing has been found in practice to present problems. In the above set out prior patent application double backed tape was identified as a preferred attachment arrangement. In practice, it has been found that contamination could result from such use. The present application solves this problem with a heat bonding of the preferred nitrocellulose support to a plastic backing. In that heat bonding process a bonding or welding of materials will take place under controlled conditions of pressure and temperature to not effect the physical characteristics of the nitrocellulose as could effect the binding of DNA thereto. While the precise nature of the protein nitrocellulose binding is not fully understood, it would seem to be the result of a number of different factors including hydrophobic and ionic interactions as well as hydrogen bonding and therefore the heat bonding process must be such as to induce a melting and flow of the support and backing without a disruption of the ability of the nitrocellulose to accept a protein bonding thereto. This heat bonding process is preferred in the present invention to construct a nitrocellulose supported with a plastic backing as a "stick support", the nitrocellulose support to receive dots of certain antigens and controls dried thereon.

As set out above, both the cited prior invention and the present invention each relate to protein dot blotting systems classified as an immunochemical technique and more specifically an enzyme-linked immunosorbent assay (ELISA). Over the past several years protein blotting onto a solid support has been increasingly applied to provide tests for solving a diverse range of problems. Such problems have involved the identification of DNA and RNA binding proteins, glycoproteins in addition to the screening of various antigens and antisera. Additionally, it has been common to utilize nitrocellulose membranes for protein blotting from fractionated proteins taken from gel matrices, and recently nitrocellulose has been used for spotting protein samples directly thereon. This procedure is generally referred to as protein spotting or, as in the present invention, dot-immunobinding and is distinct from the earlier protein blotting from a gel matrices in that in such spotting procedures a measured aliquot of protein suspension is applied directly to the surface of the membrane and allowed to absorb into the membrane. In contrast, protein blotting from a gel matrices involves placing the fractionating gel matrices containing the immobilized proteins in direct contact with the nitrocellulose membrane and allowing transfer of proteins from the gel matrices to proceed by capillary action and often require a great deal of time of several hours to several days.

The dot-immunobinding technique of the present invention consists of applying a measured amount as a dot of protein solution onto the surface of the nitrocellulose membrane. Thereafter, such membrane is preferably subjected to a blocking reaction where an agent is applied thereto to block protein binding for proteins other than those being tested for. After blocking, the spotted membrane will be used for detection of a specific antibody by subjecting the membrane to antigen-antibody reaction conditions. A discussion of earlier antibody detection procedures is set out in the earlier cited patent application Serial No. 857,643.

Both the present invention and the above-cited earlier application are directed to protein dot-immunobinding techniques. They each involve a unique utilization of an immobilized nitrocellulose membrane in the form of a dip stick or stick support. They differ from other ELISA methods in that the colored products identifying a positive reaction are insoluble rather than soluble, and each is capable of testing whole blood. Additionally, unique to the present invention, the stick support configuration of the present invention allows for greater ease with which the immunochemical reactions may be executed including the handling of the membrane. With closely controlled heat bonding of the preferred nitrocellulose membrane to a backing, the finished stick support will not include contamination as was possible with the adhesive bonding process as was formerly practiced. Further unique, the present invention provides a system that, like the earlier invention, allows for very short incubation periods of the stick support in a reagent, as opposed to an earlier standard of thirty (30) minutes. Also unique, the present invention includes a use of reagents that have been tabletized that are to be reconstituted when ready for use and accordingly have a much longer shelf life than do liquid reagents. The present invention also utilizes an improved "stick" holder and vessel structure for use therewith that are unique. And finally, the system of the present invention employs an improved washing step between incubations.

Accordingly, the present invention provides a more efficient and reliable blood serum testing system for use in the setting of a doctor's office to test, in a single procedure, for the presence of antibodies to a number of different antigens.

## SUMMARY OF THE INVENTION

It is a principal object of the present invention in an improved solid phase enzyme immunoassay test apparatus and process for detecting antibodies to provide an apparatus and procedure for accurately and reliably detecting the presence of a variety of specific antibodies in a clinical specimen within a relatively short period of time.

Another object of the present invention is to provide, as the test apparatus, a porous membrane that is mounted by heat bonding onto a rigid support and whereto dots of specific antigens are separately immobilized that will, after sequential incubations in different immunochemical reagents, with washes between, individually react in the presence of a specific antibody by changing color, thereby visually indicating the presence of such specific antibody.

Another object of the present invention is to provide long life tabletized reagents that are preferably activated by an addition of a light NaCl solution to provide a reconstituted reagent wherein are performed the sequential incubation steps.

Still another object of the present invention is to provide a vortex wash apparatus for use in a wash step between incubations.

Still another object of the present invention is to provide an improved apparatus and procedure for simultaneously detecting, in a short period of time, the presence of specific antibodies in a clinical specimen in the setting of a medical doctor's office.

The present invention is an improved enzyme-linked dot blot immunoassay technique for simultaneously detecting the presence of antibodies to several different antigens from a clinical specimen. The improved apparatus of the invention includes a porous membrane, preferably nitrocellulose, that is immobilized by heat bonding it onto a plastic support. In such heat bonding, the contact pressure and temperature on the nitrocellulose and plastic over time are closely controlled to just induce a flow therebetween, producing a welding of the layers together without causing a change in the physical characteristics of the nitrocellulose. Prior to heat bonding the backing will have had holes formed at intervals therein that serve as windows, exposing the porous nitrocellulose membrane therethrough. After bonding, an array of purified antigens are dot blotted and dried onto the porous nitrocellulose membrane, each approximately centered in one of the separate windows or compartments. A stick support mounting the immunodotted immobilized membrane is thereby formed to be held on one end for use in dip stick fashion. So arranged, the stick support is to be sequentially immersed in certain reagents with light NaCl washes between for detecting, as color changes on the purified antigen dots, antibodies from clinical samples. To provide this detection, three five (5) minute incubation steps are required. All of which incubation steps are performed at ambient temperature. The sequential incubations are performed in three separate containers that individually receive tabletized reagents. The reagents are each preferably activated by introduction of a NaCl solution thereto. Each vessel to receive, in turn, the nitrocellulose membrane end of the stick support inserted therein, whereafter the stick support membrane end is subjected to a vortex wash.

In a practice of the procedure of the present invention the first container is to contain a sample specimen diluent in tablet form that is activated or reconstituted by mixing a NaCl solution therewith along with a drop of either plasma, serum, or whole blood from a clinical specimen. The stick support is inserted into this diluent and sample for a five (5) minute incubation period during which time certain antibodies as are present in the clinical sample will bind to certain of the antigen spots. Following a fitting of the stick support into a sealed vortex tube containing a light NaCl solution, and performing a vortex type wash thereon, the stick support is inserted into a second container. The second container preferably contains, in tabletized form, an alkaline phosphatase conjugated anti-human immunoglobulin G. Prior to stick support insertion this tablet is reconstituted by introduction and mixing of a NaCl solution therewith. During this incubation step enzyme conjugate binds to antibodies as were absorbed from the clinical sample bound during the first step. Following another vortex wash utilizing the vortex tube containing the same or a new NaCl solution the stick support membrane end is immersed in a third and final container that preferably contains chromogenic substrates for the alkaline phosphatase in tabletized form that is reconstituted by mixing a preferred volume and concentration of a NaCl solution. With the stick support membrane end immersed therein the alkaline phosphatase enzyme converts the soluble chromogenic substrates into colored insoluble products than bind to the porous nitrocellulose membrane at the site of the bound enzyme conjugate. The bound products are thereby each revealed as a colored spot on the nitrocellulose membrane surface. The absence of a colored spot indicates the absence of the particular antibody in the clinical sample.

In practice, the improved enzyme-linked dot blot assay application of the present invention has been found to consistently, accurately and simultaneously identify the presence of antibodies to a variety of antigens in a single procedure that can be practiced in a short period of time.


BRIEF DESCRIPTION OF THE DRAWINGS

These and other objects and features of the present invention in an improved solid phase enzyme immunoassay test apparatus and process for its use for detecting antibodies to specific antigens in a clinical specimen will become more fully apparent from the following description in which the invention is described in detail in conjunction with the accompanying drawings.

Fig. 1 is an exploded view of a section of the nitrocellulose membrane aligned to a plastic support showing arrows to illustrate the application of controlled heat and pressure thereto, bonding the membrane and support together;

Fig. 2 is an exploded perspective view of the bonded membrane and support of Fig. 1 as the stick support, the support shown to have holes punched at intervals therethrough, the nitrocellulose membrane surface is exposed to receive, as separate blocked dots, an array of purified antigens immobilized thereon;

Fig. 3 is a flow schematic showing the steps involved in practice of the procedure of the present invention with the stick support of Fig. 2 for determining, by color change to the individual dots, the presence of antibodies to specific immobilized antigens present in a clinical specimen.

Figs. 4A through 4C show the stick support of Fig. 2 being mounted and installed in a vortex wash tube for vortex washing; and

Fig. 5 is a view of a vessel for use in one of the incubation steps of Fig. 3 showing with arrows the introduction and mixing of a tabletized reagent and NaCl activating solution.

DETAILED DESCRIPTION OF THE INVENTION

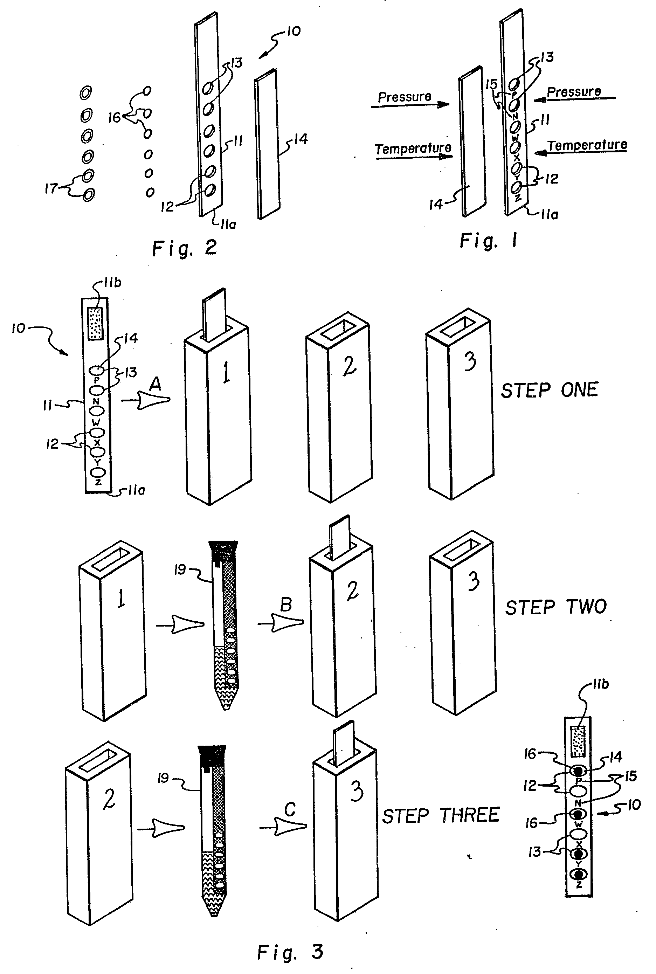

In the present invention specific antibodies to several different antigens can be simultaneously detected in a test procedure from a single clinical specimen. Shown in Fig. 2, the test procedure preferably employs a stick support 10 whereon are arranged an array of dot-immunobound purified antigens. The stick support 10 includes, as shown in Fig. 1, a support 11 that is preferably a long rectangular thin wafer of rigid plastic of approximately 40 mil thickness with an array of round or ellipsoid windows 12 formed at spaced intervals. The windows 12 are preferably beveled inwardly at 13 to approximately fifteen degrees from a right angle and are arranged at intervals from a lower end 11. The individual windows are each shown labeled with characters below that window as are appropriate to identify the particular antigen dot fixed in the window above.

A porous membrane 14, preferably nitrocellulose, is attached by heat bonding as illustrated in Fig. 1, to support 11 to form the stick support 10. In this heat bonding process support 11 and membrane 14 are pressed together for a short period of time at a pressure and temperature that are closely controlled to introduce a slight melting of the junction of each of the surfaces. This melting is just sufficient to provide a welding together of the layers without a disruption of the nitrocellulose membrane physical characteristics as could effect the ability of the nitrocellulose to serve as a transfer membrane. In practice, to provide a preferred heat bonding, the layers are subject to temperature of approximately two hundred twenty degrees Fahrenheit, plus or minus twenty degrees Fahrenheit, and a pressure of approximately thirty (30) pounds per square inch, plus or minus three (3) pounds per square inch for approximately one (1) second plus or minus point two (0.2) seconds, for a nitrocellulose membrane layer and polyvinylchloride support. Should, however, another membrane material or different plastic support be utilized, the preferred heat bonding temperature and pressure would have to be adjusted for the particular material or materials.

Characters 15, shown as P, N, W, X, Y, and Z, in Figs. 1 and 2, are preferably formed to the support 11, positioned on line underneath each window 12 for identifying the particular window or dot therein. A roughened surface is preferably provided on a stick support handle end 11b, as shown best in Fig. 2, to provide a surface for receiving writing or the like thereon allowing an operator to mark the particular stick support 10, for clinical sample identification.

Once stick support 10 has been assembled by heat bonding the porous membrane 14 to the support 11, the portions of that porous membrane that show through the individual windows 12 are spotted (immunodotted) with approximately one microliter each of an antigen sample, shown as dots 16. It should, of course, be understood that volumes other than one microliter per dot may be employed within the scope of this disclosure. The different samples are dispensed as dots 16 into the center of each of the lower windows 12 identified with characters W, X, Y, and Z. Thereabove, a second window from the top is shown labeled as N. This window preferably contains a negative control. Such control as is spotted in this window 12 consists of a buffer solution as is used for the solubilization/dilution of all the other samples that are spotted in the lettered windows, or can be a negative control antigen such as a cellular extract of uninfected cells as used for viral antigen production. Finally, a top window identified as P, adjacent to stick support end 11b can be allocated for receiving a positive control. The positive control is to verify that all reagents are functioning properly and that the test procedures have been properly executed. Such control will also verify that the three reaction vessels, shown in Fig. 3, are sufficiently reconstituted to the desired volume to successfully perform the test procedure. In practice, this window has been spotted with a solution of purified human immunoglobulin G (IgG). It should, of course, be understood that solutions other than those described may be used as the positive and negative controls or these controls may be omitted entirely and the windows designated as P and N could be dispensed with or used for other purposes within the scope of this disclosure. The windows 12, identified at W, X, Y, and Z, in practice, are preferably spotted with various purified antigens that are each specific for a type of antibody to be tested for. The total number of types of antibodies to be tested for, of course, determines the number of windows 12 as are needed. In practice, as set out in the examples below, a stick support 10 that incorporates six windows, two for the positive and negative controls with the other four windows spotted with various purified antigens, has been used successfully.

Once the porous membrane 14 surface showing through windows 12 has been spotted with the appropriate solutions these spots 16 are allowed to air dry at ambient temperature. Such drying usually requires several minutes though longer drying times may be employed without noticeable effect. Also, temperatures other than ambient may be employed without noticeable effect.

Following drying, the nitrocellulose membrane-bound end 11a of the stick support 10 is blocked or capped, as illustrated in Fig. 2 by the centrally holed disks 17 that represent coating of the membrane. Blocking is employed to occupy the nitrocellulose with a non-interfering protein, or other like compound that will prevent the non-specific binding of the immunochemical compounds thereto. This blocking is such as to minimally effect the spots 16, and is practiced to limit the high affinity that most porous membranes, such as nitrocellulose, exhibit to non-specific absorption of a wide range of proteins. In the present invention, to provide this blocking, the membrane-bound end 11a of the stick support 10 is preferably immersed in a solution containing non-fat dry milk in a Tris-saline solution (5% non-fat dry milk in 10mM Tris, pH 7.4, 0.9% NaCl) for approximately fifteen (15) minutes at ambient temperature. Other blocking solutions, however, may be employed to include solutions of albumin, caesin, gelatin, detergents, amino rich compounds, and others. Also, the time and temperature that the membrane is exposed to the blocking agent may be varied depending on the membrane employed and the desired level of acceptable sensitivity/interference. Further, blocking may be omitted entirely for some applications where a lower level of sensitivity is acceptable.

Following blocking, stick support 10 is briefly rinsed in distilled water and is allowed to air dry at ambient temperature. Other rinse solutions may also be employed such as buffered or detergent containing solutions where greater levels of sensitivity for a particular test configuration are desired. Blocked stick supports 10 can be packaged in foil pouches and stored at refrigerator temperature until use, or they may be packaged in other suitable containers or not packaged at all depending on their anticipated use. Packaged or unpackaged stick supports 10 may also be stored at ambient temperature for some applications. Further, while not shown, two such stick supports 10 can be manufactured or arranged together in back to back configuration to double the number of specific antibodies that can be tested for in a single procedure.

Fig. 3 illustrates a preferred practice of the process of the present invention as including successive incubation steps of the stick support 10 of Figs. 1 and 2 in containers 1, 2 and 3 with rinses between incubations. To detect a specific antibody from a clinical sample a stick support 10, prepared as set out above, is successively incubated at ambient temperature for five (5) minutes in each of three separate immunochemical solutions that are contained, respectively, in containers 1, 2 and 3, the stick support also shown as being rinsed, preferably in a light salt (NaCl) solution, between each incubation. While the steps shown in Fig. 3 are preferably performed under ambient conditions other incubation temperatures and times may also be employed to effect a speeding up or slow down of an incubation within the scope of this disclosure.

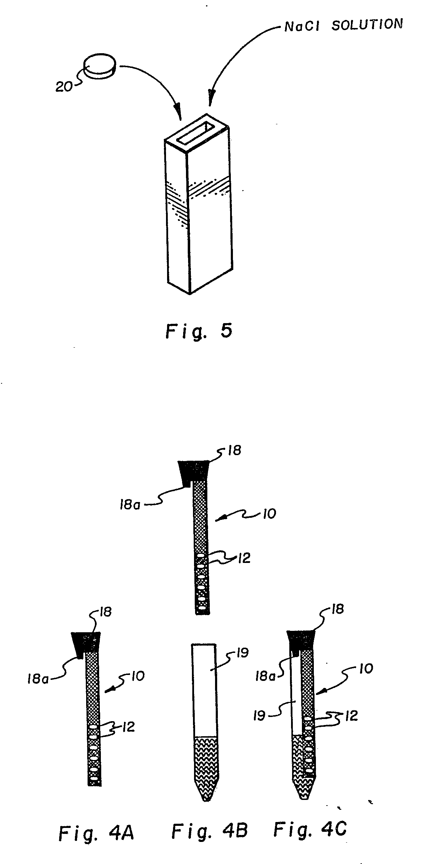

The process of the present invention is preferably performed in small plastic containers, each to contain an immunochemical solution. Each container is to receive the nitrocellulose bound membrane end of the stick support 10 fitted therein. The containers 1, 2 and 3 are preferably empty prior to its receiving a tablet 20 of immunochemical reagents dropped therein, as illustrated in Fig. 5. Preferably, each tablet is a 100 mg tablet, that is added to the empty dry container and, preferably is reconstituted with 2 ml of 0.1 M NaCl solution. Prior to the immersion of the stick support membrane end therein, a disposable plastic pipette, not shown, may be used to stir the contents of each vessel to insure the dissolution of the individual tablet to provide adequate mixing of the resuspended reagents, the pipette is moved so as to force the liquid contents of each container up and down several times as needed.

The reagent tablets are prepared from the materials described below. In that preparation appropriate absorbed materials are dried and then blended with the other chemical reagents. The blends are then tabletized according to standard tabletizing procedures common to the tabletizing industry to include mixing and compression molding or stamping. The tablets are prepared from bulk quantities of the chemical reagents as listed below which recipes will each produce approximately one one hundred mg tablet of each reagent. Of course, in practice the tablets are preferably compounded in large batches and the weights of materials are accordingly increased proportionally for the number of tablets to be produced. Additionally, a variety of modifications of the reagent recipes are possible within the scope of this disclosure. Such modifications may be employed to enhance tablet disintegration time, stability, cost, and activity of the critical immunochemical reagents. Because of their shelf life, packaging and handling ease and low production cost tabletized immunochemical reagents are more desirable than lyophilized reagents.

The preferred constituents of the tablets for each vessel are as follows:

### Tabletized Sample Diluent

#### Vessel 1

37 mg cellulose
2.647 mg Tris HCl
0.387 mg Tris Base
40 mg non-fat dry milk
19 mg NaCl
1 mg magnesium stearate

### Tabletized Conjugate

#### Vessel 2

29.24 mg cellulose
3.408 mg conjugate
13.696 mg Tris HCl
1.664 mg Tris Base
0.24 mg magnesium sulphate
46.752 mg NaCl
4 mg bovine serum albumin
1 mg magnesium stearate
(The conjugate may be either lyophilized conjugate or liquid conjugate absorbed separately onto the cellulose and allowed to dry)

### Tabletized Enzyme Substrate

#### Vessel 3

27.67 mg mannitol
33.82 mg cellulose

0.66 mg nitro blue tetrazolium (NBT)
0.3333 mg 5-bromo-4-chloro-3-indolyl phospophate (BCIP)
1.68 mg Tris HCl
22.8 mg Tris Base
12.04 mg magnesium sulphate
1 mg magnesium stearate

(The NBT is dissolved in 0.0088 ml 70% dimethylformamide and absorbed separately onto the cellulose and allowed to dry. The BCIP is dissolved in 0.006667 ml dimethylformamide and absorbed separately onto the mannitol and allowed to dry.)

Shown in Fig. 3, the first of the three containers, numbered 1, preferably contains a clinical sample diluent that is provided to dilute the clinical sample provided by a patient to a less concentrated form and will also provide blocking agents to minimize non-specific binding of the clinical sample to the membrane. The preferred reconstituted diluent for vessel 1 is as set out above. Additionally, within the scope of this disclosure, other solutions may also be employed as diluent such as, for example, Tris-saline, phosphate buffered saline, detergent containing solutions, and the like, providing such other solution minimizes non-specific interactions of the immunochemical reagents.

After the tablet containing the constituents of the diluent is reconstituted, as illustrated in Fig. 5, utilizing the preferred 2 ml of 0.1 M NaCl solution, a drop of either serum, plasma, or whole blood is added to that container 1 and the container constituents are mixed thoroughly. In practice, it has been found that a very small or very large drop of blood or several drops of blood may be added to the sample diluent without affecting test results. (An average drop is approximately 45-50 microliters). Where several drops of whole blood are desired for a particular application, coagulation problems may be avoided by employing the diluted blood sample with relative haste such that the sample will have been used and discarded prior to the onset of coagulation. Alternatively, an anti-coagulant may be included as part of the reagent or added to the reconstituted diluent. During this first incubation step, if present, a specific antibody from the clinical sample will bind to the spot of that specific antigen.

The above step 1 includes incubating the stick support 10 in the presence of the clinical sample and sample diluent, illustrated as arrow A. After an incubation for approximately a five (5) minute period the stick support is removed from the container 1 and briefly rinsed. The rinse is preferably performed by fitting the stick support in a vortex tube 19, shown in Fig. 3, step 2. To provide for this wash step, shown in Fig. 4A the stick support 10 top end is fitted by sliding it into an appropriate slit cut laterally in an undersurface of a stopper 18 that is preferably formed of neoprene polypropolene, or a like material. The edges of that lateral cut are to flex apart, allowing passage of the stick support end to fit therebetween and then flex back to grip that stick support end. In alignment with that slit is shown a pier 18a whereagainst one end edge of the stick support end will come to rest. So arranged, the other stick support edge will essentially align with the neoprene stopper circumference. As shown in Fig. 4B, an operator can thereby align and lower the stick support 10 mounted to stopper 18 into a vortex wash tube 19 that is partially filled with a rinse solution, preferably a 0.1 M NaCl solution, or the like. Fig. 4C shows the stopper mounted stick support 10 inserted into the vortex wash tube 19, with the stopper 18 at its outer circumference flexed against to seal over the tube open end. The stick support 10 is positioned, as shown in Fig. 4C, such that the windows 12 will face towards the person performing the test. Preferably, as illustrated in Figs. 4A through 4C, the stick support is subjected to a vortex rinse in a 0.1 M NaCl solution between each incubation (performed in vessels 1 and 2). Optionally, this wash step can also be performed after a last incubation (performed in vessel 3). In each such wash, the stick support 10 is positioned, as shown in Fig. 4C such that the one edge butts against the vortex tube 19 wall, with the stopper 18 holding the stick support in this position and sealing over the tube end. A vortex wash is preferred for all rinse steps and is to provide an acceptable level of sensitivity/interference of the now activated dots 16, which wash may, but need not, be performed in the same wash solution.

In the rinse step between the incubations in vessels 1 and 2, the operator holds the vortex tube 19 vertically with it positioned in a vortex machine while the strip support 10 is vortex washed for a minimum of ten (10) seconds using a maximum vortex speed. In practice, machines known as Vortex-Genie, model K-550-G and Vortex Genie-2, model G-560 manufactured for Fisher Scientific by Scientific Industries, Inc., Bohemia, N.Y. have been found to operate effectively to perform this wash step.

In the washing step, it is important that the stopper mounted strip support be relatively free of vibration. While vortexing the vortex tube 19 therefore, it is helpful for the operator to hold the tube between the thumb and third finger with the index finger placed firmly on top of the stopper 19. Should the strip support 10 begin to vibrate back and forth during the vortex washing the index finger may be used to apply a slight downward pressure on the stopper so as to urge the slit edges closer together, clamping against the stick support end, to prevent further vibration.

As desired, the neoprene stopper 19 may be replaced with another like device provided such other device can be used to perform the same function, that of retaining the antigen dotted membrane of the stick support firmly in place during the vortex washing process. Also, the rinse or wash solution may be varied both as to volume and composition. It is, however, important that NaCl be used in the solution though a variety of concentrations are adequate, and a solution of 1 M - 0.1 M NaCl solution is preferred. Such NaCl solution, it has been found, enhances the intensity of the immunochemical reactions thus permitting a more intense visual indication upon completion of the test. The vortex washing procedure is desirable in that it is highly reproducible, rapid, produces an enhanced immunochemical reaction sensitivity, utilizes a minimum volume of washing solution, and has consumer appeal.

Following the first rinse, arrow B in step 2, the stick support 10 is inserted in container 2. This container preferably contains a reconstituted conjugate preferably produced from mixing approximately 2 ml of 0.1 M NaCl solution with the tabletized conjugate. A preferred conjugate is as set out above. However, conjugates of other specificities, it should be understood, are also within the scope of the present invention. Use of an enzyme conjugate essentially provides an enzyme-linked immunosorbent assay (ELISA) based procedures as set out in the above-cited earlier patent application of three of the present inventors, and so will not be further discussed herein. During this incubation of the stick support 10 in container 2, the enzyme labeled conjugate will bind to clinical sample antibodies that have bound to specific antigen spots from the first incubation in container 1. Additionally, the conjugate will also bind to the positive control spot (preferably human IgG) during this incubation.

The incubation of the stick support 10 in vessel 2 is followed by another rinse, that is illustrated as the vortex tube 19 shown in step 3 before arrow C of Fig. 3. Preferably, this wash is conducted essentially the same as the vortex washing procedure set out hereinabove. In this wash step the wash solution used in the first wash can be reused, or a fresh wash of NaCl solution may be used. Also, as the presence of NaCl in this second wash solution is not critical, it may be omitted without effect and distilled water used in its place. It is, however, desirable to reuse the first wash solution as it minimizes the quantity of reagents to be packaged and simplifies the test procedure.

Following the second vortex wash, the stick support 10 is installed into the third container that contains enzyme substrates for the enzyme conjugate that will react with the conjugate in such a way as to visually reveal the presence of said conjugate. The preferred enzyme substrate is that shown above and is reconstituted by mixing that enzyme substrate in its tabletized form with approximately 2 ml of 0.1 M NaCl solution. It should, however, be understood that other enzyme substrates may be substituted within the scope of this disclosure so long as such would produce a visible color change to a particular dot 16 on the stick support nitrocellulose membrane. During the incubation period the preferred alkaline phosphatase will interact with the NBT/BCIP substrates. Shown as the product of step 3, that is a result of this enzymatic interaction, is the production and deposition of insoluble colored precipitates on the nitrocellulose membrane 14 at the site of the bound enzyme conjugate dot 16. This deposition and its tight binding to the membrane produces on the stick support 10 a blue-violet colored spot 16 against a white background of the nitrocellulose membrane 14 within the ellipsoid window 12. This incubation may, but need not, be followed with a brief rinse that is again preferably a vortex wash in NaCl solution or distilled water.

Colored spots that have appeared within one or more of the windows after the third incubation indicate the presence of a specific antibody for that specific antigen in the clinical specimen, with the absence of such colored spot indicating that such specific antibody is not present. Further, as a verification of the validity of the test results, examination of the positive and negative control windows P and N should show a presence of a colored spot in the preferred positive control window with no detectable spot in the preferred negative control window. Results other than these for the two controls invalidates the test. This is because a color change of the positive control and no color change of the negative control will indicate that all test reagents worked properly at the time of testing and that the proper procedures for executing the test were followed.

In the earlier above-cited patent application certain examples were set out showing the operation of the test procedure and results. Reference is hereby made to these examples and to the prior application as a whole as a demonstration of the utility and practicality of this procedure and therefore these examples will not be further set out herein.

The present invention and the dot blot system of the prior application for testing for the presence of one or more antibodies has been found in practice to have several advantages over earlier serological procedures. Specifically, that: the test can simultaneously identify the presence of antibodies to several antigens; the test can be performed using a single drop of whole blood; the test does not require highly trained personnel and sophisticated instrumentation; the test can be done very rapidly, in less than twenty (20) minutes; the test preferably includes its own built in positive and negative controls; the test is easily read; and, the tested stick support can be kept as a permanent record.

Specific embodiments of the present invention are as follows:

1. A solid phase enzyme immunoassay system for visually detecting specific antibody presence in a clinical sample comprising, a chemically neutral plastic backing member wherethrough a plurality of openings have been formed; a porous nitrocellulose membrane heat bonded to said plastic backing member at a temperature and pressure so as to create a liquification and running together of the materials at their junction, creating a stick support, sections of said porous nitrocellulose membrane exposed through said plastic backing member openings for receiving and maintaining the individual integrity of dots of select purified antigens blotted thereon; individual dots of select purified antigens blotted on said porous nitrocellulose membrane one in each of said openings; vessel means for sequentially receiving and incubating said porous nitrocellulose membrane end of said stick support in immunochemical solutions that are each reconstituted from tabletized immunochemical constituents, each tablet reconstituted by adding therewith approximately a 0.1 M NaCl solution mixed within said vessel means, a first vessel means to contain a clinical sample diluent whereto a small amount of plasma serum or whole blood of approximately one drop is added, the second vessel means to contain an enzyme conjugate that will bind to a specific antibody as may be present in the clinical sample and to a specified purified antigen fixed as a dot to said nitrocellulose membrane, and a third vessel means to contain an enzyme conjugate substrate to interact with the enzyme conjugate that has bound said specific antibody to said dot of specified purified antigen so as to produce a visual color change at that dot; and a means for vortex washing said porous nitrocellulose membrane end of said stick support between incubations.

2. An immunoassay system as described in Embodiment 1 further including forming markings in the plastic backing member, one adjacent to each opening for identifying a specific purified antigen dot immobilized on the porous nitrocellulose membrane surface exposed through that opening, which openings are formed at spaced intervals; and the stick support includes a finger engaging portion consisting of a roughened section also for writing thereon on one end thereof.

3. An immunoassay system as described in Embodiment 1 wherein the heat bonding of the plastic backing member with the porous nitrocellulose member is at a contact pressure of thirty (30) pounds per square inch plus or minus three (3) pounds per square inch and at a temperature of two hundred twenty degrees Fahrenheit plus or minus twenty degrees Fahrenheit for one (1) second plus or minus point two (0.2) seconds.

4. An immunoassay system as described in Embodiment 1 wherein six aligned openings are formed at spaced apart intervals longitudinally in said plastic backing member each to expose a section of porous nitrocellulose membrane therein, a lower four openings to each receive a specific purified antigen dot immobilized on said section of said porous nitrocellulose membrane exposed therein, the remaining two upper windows receiving, respectively, a dot each of positive and negative controls immobilized thereon.

5. An immunoassay system as described in Embodiment 4 wherein the positive control is a dot of purified human immunoglobulin G (IgG) that will exhibit a color change after the sequential incubations in the immunochemical solutions so as to indicate that the test reagents are working properly and that proper test procedures have been followed; and the negative control is either a dot of a buffer solution appropriate for the solubilization/dilution of the antigen dots spotted on the other windows or a negative control antigen such as a cellular extract of uninfected cells as used for viral antigen production, which negative control to remain unchanged with the sequential incubations in said immunochemical solutions so long as the test reagents are working properly and the proper test procedures have been followed.

6. An immunoassay system as described in Embodiment 1 further including blocking the area of the porous membrane section in each opening with a non-interfering protein for preventing non-specific binding of immunochemical reagents.

7. An immunoassay system as described in Embodiment 1 wherein a sample diluent is a buffer selected to minimize non-specific interactions of the immunochemical reagents that has been tabletized and is reactivated in the first vessel means by introduction thereto and mixing with approximately 2 ml of a 0.1 M NaCl solution.

8. An immunoassay system as described in Embodiment 1 wherein the enzyme conjugate is one selected to minimize non-specific interactions that has been tabletized and is reactivated in the second vessel means by introduction thereto and mixing with approximately 2 ml of 0.1 M NaCl solution.

9. An immunoassay system as described in Embodiment 1 wherein the enzyme conjugate substrate is one that will react to the selected enzyme conjugate to produce a detectable color change at the antigen dot.

10. An immunoassay system as described in Embodiment 1 wherein the dotted exposed sections of porous nitrocellulose membrane between incubations are rinsed by mounting the stick support in a vortex tube means that contains a 0.1 M NaCl solution, said vortex tube means to be supported in a vortex machine means that is operated to perform a vortex wash on said porous nitrocellulose membrane portion of said stick support.

11. A process for visually detecting specific antibody presence in a clinical sample comprising the steps of, immobilizing dots of specific purified antigen blotted at intervals on a porous nitrocellulose membrane that is backed with plastic backing; performing successive incubations of the porous nitrocellulose membrane mounting the antigen dots in select immunochemicals that are each reconstituted from a tabletized state by mixing a 0.1 M NaCl solution therewith, a first incubation of said antigen dots taking place in a sample diluent consisting of buffers whereto has been added a small amount of plasma, serum or whole blood of approximately one drop to form a clinical sample, said diluent selected to dilute the sample while minimizing non-specific immunochemical interactions as would interfere with desired chemical reactions; a vortex wash of said porous nitrocellulose membrane whereon said antigen dots are immobilized in a 0.1 M NaCl solution; a second incubation of said antigen dots in an enzyme conjugate that is suitable for performing an enzyme-linked immunosorbent assay (ELISA) based procedure with said conjugate to bind to the clinical sample antibodies as have bound to certain of said specific antigen spots on the porous nitrocellulose membrane: a vortex wash of porous nitrocellulose membrane wherein said antigen dots are immobilized in a 0.1 M NaCl solution or distilled water; and a third incubation of said antigen dots in an enzyme conjugate substrate of the enzyme of the second incubation selected to produce a detectable dot color change.

12. A process as described in Embodiment 11 plastic backing member is provided with a plurality of longitudinally equally spaced apart openings; and the porous nitrocellulose membrane is heat bonded thereto or a temperature and pressure applied for a time period to produce a welding together of the materials without changing the characteristics of said nitrocellulose membrane, a section of said porous nitrocellulose membrane exposed through each said opening, the porous membrane section in each said opening to receive a dot of a specific purified antigen spotted and dried thereon.

13. A process as described in Embodiment 12, wherein, additional to the spots of specific purified antigen, in certain of the openings the porous nitrocellulose membrane is spotted with positive and negative controls, which positive control is a dot of human immunoglobulin G (IgG) that will exhibit a color change after the sequential incubations in the select immunochemical solutions, the color change indicating that the chemicals are working properly and that proper test procedures have been followed, and the negative control is a dot of a buffer solution appropriate for the solubilization/dilution of the antigen dots spotted in the other openings the negative control to remain unchanged with the sequential incubation in said reconstituted immunochemical solutions indicating that the chemicals are working properly and that proper test procedures have been followed.

14. A process as described in Embodiment 13 further including blocking the area of the porous nitrocellulose membrane section in each opening with a non-interfering protein for preventing non-specific immunochemical binding.

15. In this Embodiment, the numbers in brackets refer to reference numerals in Figures 1 to 5.

An improved test apparatus and process for its use to simultaneously detect the presence of a variety of specific antibodies in a clinical specimen, wherein the process involves an enzyme-linked dot blot immunoassay where an array of specific antigens that are used to detect specific antibodies are separately immobilized as dots (16) on a porous membrane (14) that is preferably porous nitrocellulose which is itself immobilized by heat bonding on a rigid support (11). The immobilized nitrocellulose membrane is used in the form of a stick support (10).

Specific antibodies are detected by sequential incubations of the antigen stick support in three different solutions of immunochemical reagents that are reconstituted with a 0.1 M NaCl solution from tabletized constituents. The stick support (10) is subjected to a vortex wash in a vortex tube (19), containing a light NaCl solution, between incubations and, after the final incubation, the presence of specific antibody is evidenced by the appearance of a blue-violet colour change to a dot of a specific antibody immobilized on the porous nitrocellulose membrane with the absence of colour development indicating the absence of such specific antibody.

While a preferred embodiment of the invention in an improved test apparatus for detecting antibodies in a clinical specimen and process for its use have been shown and described herein, it should be apparent that this disclosure is made by way of example only and that variations to the apparatus and process are possible within the scope of this disclosure without departing from the subject matter coming within the scope of the following claims, which claims we regard as our invention.

**Claims**

1. A method of preparing a stick component for a solid phase enzyme immunoassay system or kit which comprises heat bonding a porous nitrocellulose membrane to a chemically neutral plastic backing member comprising a plurality of openings, which backing member extends beyond the nitrocellulose membrane at its upper end when the two are bonded together to form a stick for handling, and applying a specific purified antigen dot to the nitrocellulose through at least one of the openings.

2. A method according to Claim 1 wherein the heat bonding is carried out at a contact pressure of 207 ± 21 kPa (30 ± 3 psi) and a temperature of 104 ± 11°C (220 ± 20°F) for 1 ± 0.2 seconds.

3. A method according to Claim 1 or 2 which comprises subjecting the nitrocellulose membrane to a blocking reaction after application of the antigen, to prevent non specific binding to the antigen by adventitious protein.

4. A method according to any one of the preceding claims wherein a positive and a negative control are applied to the nitrocellulose through at least two of the openings, respectively, the positive control being, for example, a dot of human immunoglobulin G (IgG) which exhibits a colour change when the component is in use and the negative control being, for example, a dot of buffer solution appropriate for the solubilisation/dilution of the antigen dots applied to the membrane through the other openings, which remains unchanged in colour when the component is in use.

5. A method according to any one of the preceding claims which includes marking the plastic backing member next to each opening to identify the specific antigen dot applied to the nitrocellulose through each opening; and forming a roughened section on the end of the plastic backing member extending beyond the nitrocellulose membrane to create a finger engaging portion and a section suitable for writing on.

6. A solid phase immunoassay system or kit for the visual detection of specific antibodies in a chemical sample, comprising a component prepared by the process as claimed in any one of Claims 1 to 5, at least one vessel into which the component can be inserted for incubation, at least one immunochemical constituent in tablet form, which tablet is reconstituted by the addition of a 0.1.M NaCl solution, and a means for vortex washing the nitrocellulose membrane end of the component.

7. A solid phase immunoassay system or kit according to Claim 6 wherein the immunochemical constituents comprise a sample diluent, such as a buffer selected to minimise non specific interactions of the immunochemical reagents; an enzyme conjugate such as one that will minimise non specific interactions; and an enzyme conjugate substrate, such as a substrate that will produce a colour change at the antigen dot by reaction with the enzyme conjugate.

8. A method for the visual detection of specific antibodies in a clinical sample comprising the steps of applying dots of specific purified antigen at intervals on a porous nitrocellulose membrane bonded to a plastic backing and performing successive incubations of the porous nitrocellulose membrane by mounting the antigen dots in select immunochemicals that are each reconstituted from a tabletized state by mixing a 0.1 M NaCl solution therewith.

9. A method according to Claim 8 which comprises a first incubation of the nitrocellulose membrane in a sample diluent consisting of buffers to which has been added a small amount of plasma, serum or whole blood to form a clinical sample, the diluent being selected to dilute the sample while minimizing non-specific immunochemical interations; carrying out a vortex wash of the porous nitrocellulose membrane in a 0.1 M NaCl solution; performing a second incubation in an enzyme conjugate that is suitable for performing an enzyme-linked immunosorbent assay (ELISA) based procedure with said conjugate to bind to the clinical sample antibodies as have bound to certain of said specific antigen spots on the porous nitrocellulose membrane;
carrying out a second vortex wash of the porous nitrocellulose membrane in a 0.1 M NaCl solution or distilled water;
and performing a third incubation in an enzyme conjugate substrate of the enzyme of the second incubation, the substrate being such that a detectable dot colour change is produced.

10. A method according to Claim 8 or 9 wherein the porous nitrocellulose membrane bonded to a plastic backing member is a component prepared by the process as claimed in any one of Claims 1 to 5.

Fig. 2

Fig. 1

Fig. 3

STEP ONE

STEP TWO

STEP THREE

*Fig. 5*

*Fig. 4A*   *Fig. 4B*   *Fig. 4C*